(19) Europäisches Patentamt
European Patent Office
Office européen des brevets

(11) Publication number: 0 152 281
A2

(12) EUROPEAN PATENT APPLICATION

(21) Application number: 85300822.5

(22) Date of filing: 07.02.85

(51) Int. Cl.[4]: A 61 K 31/44
A 61 K 47/00, A 61 L 15/03

(30) Priority: 08.02.84 JP 21404/84
17.05.84 JP 99371/84
26.07.84 JP 156288/84

(43) Date of publication of application:
21.08.85 Bulletin 85/34

(84) Designated Contracting States:
DE FR GB IT

(71) Applicant: YAMANOUCHI PHARMACEUTICAL CO. LTD.
No. 5-1 Nihonbashi-Honcho, 2-chome Chuo-ku
Tokyo(JP)

(72) Inventor: Konno, Yutaka
No. 21-6, Bessho-cho
Ohmiya-shi Saitama(JP)

(72) Inventor: Kawata, Hiroitsu
No. 1-7, Ohaza Namiki
Kawagoe-shi Saitama(JP)

(72) Inventor: Aruga, Masayoshi
No. 16-14, Midorigaoka 5-chome
Ageo-shi Saitama(JP)

(72) Inventor: Sonobe, Takashi
No. 10-12, Fujimi 4-chome
Fukiage-cho Kitaadachi-gun Saitama(JP)

(72) Inventor: Mitomi, Mitsuo
No. 23-2, Ohyata 1-chome
Adachi-ku Tokyo(JP)

(74) Representative: Geering, Keith Edwin et al,
REDDIE & GROSE 16 Theobalds Road
London WC1X 8PL(GB)

(54) **Transdermal formulation of nicardipine hydrochloride.**

(57) A transdermal formulation of nicardipine hydrochloride which comprises [a] a transdermal formulation base compounded with [b] nicardipine hydrochloride dissolved or suspended in a liquid containing a urea and at least one compound selected from propylene glycol, monohydric alcohols having 2 to 4 carbon atoms, lactic acid, thioglycol, middle chain fatty acid glycerides and sorbitan middle chain fatty acid esters. Such formulations give improved percutaneous absorption of the nicardipine hydrochloride.

Inclusion in the formulation of crystallizing inhibitor for nicardipine hydrochloride can improve the stability of the formulation.

EP 0 152 281 A2

Croydon Printing Company Ltd

0152281

# TRANSDERMAL FORMULATION OF NICARDIPINE HYDROCHLORIDE

Recently, systemic treatment by the transdermal administration of medicaments such a nitroglycerin, scopolamine and isosorbide dinitrate has been tried. Transdermal drug administration has several advantages e.g. simplicity, prolonged efficacy, less occurence of side effects, and avoidance of inactivating passage of the drug through the liver.

Nicardipine hydrochloride is a very useful compound having cerebral vascular dilator activity, coronary dilator activity, and an anti-hypertension activity, and in view of this,effective transdermal administration would be a desirable administration method for the compound. However, skin has poor permeability to nicardipine hydrochloride (in contrast to nitroglycerin, etc.), and no effective transdermal formulation of the compound has been obtained using conventional transdermal formulation ingredients.

Since the epidermal tissue of an animal has a barrier mechanism for preventing invasion of foreign matter, the percutaneous absorption of medicaments largely depends upon the specific properties of the medicaments and is very complicated since it is determined by the interaction between the medicament, the base, and the skin. Accordingly, one cannot in general foretell, from known transdermal medicament

preparations, what formulation will promote the percutaneous absorption of a different medicament of inherently poor percutaneous absorbability.

The present invention provides a transdermal formulation of nicardipine hydrochloride which comprises [a] a transdermal formulation base compounded with [b] nicardipine hydrochloride dissolved or suspended in a liquid containing a urea and at least one compound selected from propylene glycol, monohydric alcohols having 2 to 4 carbon atoms, lactic acid, thioglycol, middle chain fatty acid glycerides and sorbitan middle chain fatty acid esters. Such formulations give improved percutaneous absorption of the nicardipine hydrochloride.

Inclusion in the formulation of crystallizing inhibitor for nicardipine hydrochloride can improve the stability of the formulation.

The invention also provides above composition [b] per se, for use in transdermal applications; such composition [b] may include said inhibitor, though the latter when used can be included after compounding of [b] with a transdermal formulation base.

Examples of the monohydric alcohols having 2 to 4 carbon atoms for use in this invention are ethanol, propanol, isopropanol, etc.

Examples of the middle chain fatty acid glycerides for use in this invention are mono- and di-esters of

glycerol and fatty acids having 6 to 12 carbon atoms; specific examples of them are the mono- and di-glycerides of caproic, caprylic, capric and lauric acids. These materials may be used individually or as a mixture of two or more thereof. For example, a mixture of 54.3% caprylic acid mono-glyceride and 37% caprylic acid di-glyceride is commercially available under the trade name "Nikkol MGK" (made by Nikko Chemicals Co.), and a product containing more than 85% caprylic acid monoglyceride is commercially available under the trade name "Sunsoft No.700p-2" (made by Taiyo Kagaku K.K.).These commercially available products can be used in this invention.

The sorbitan middle chain fatty acid esters for use in this invention include mono- and di-esters of sorbitol and fatty acids of 6 to 12 carbon atoms. Specific examples of these esters are sorbitan monocaproic acid ester, sorbitan dicaproic acid ester, sorbitan monocaprylic acid ester, sorbitan dicaprylic acid ester, sorbitan monocapric acid ester, sorbitan dicapric acid ester, sorbitan monolauric acid ester, and sorbitan dilauric acid ester. They can be used individually or as a mixture of two or more thereof.

The proportion of nicardipine hydrochloride is usually 0.5 or 1 to 2 w/w% of the total final formulation, but may vary according to the formulation form, e.g. in the case of a cataplasm the proportion of the medicament may be low. All proportions herein are by

weight of the total final formulation unless otherwise stated.

The proportion of middle chain fatty acid glyceride and/or sorbitan middle chain fatty acid ester, when used in the transdermal formulations of this invention, is suitably 0.5 to 20 w/w%, preferably 1 to 15 w/w%.

The ureas for use in this invention include urea, thiourea and the lower alkyl derivatives thereof, such as ethylurea, 1,1-dimethylurea, 1,1-diethylurea, ethyleneurea 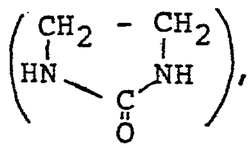, etc.

The urea is usually used as a solution or suspension in water. The proportion of the urea (dry wt.) in the transdermal formulations of this invention is e.g. 0.3 to 30 w/w%, preferably 1 to 10 w/w%.

The proportion of monohydric alcohol and/or thioglycol, when used in the transdermal formulations of this invention, is e.g. 0.3 to 30 w/w%, preferably 1 to 10 w/w%.

The proportion of lactic acid, when present, is e.g. 0.3 to 10 w/w%, preferably 0.5 to 5 w/w% of the formulation.

Propylene glycol can be present in the transdermal formulations of this invention in a proportion of e.g. 0.5 to 15 w/w%, preferably 1 to 10 w/w%. Propylene glycol increases the solubility if nicardipine hydrochloride and has humidity retaining and antiseptic

actions, as well as promoting the percutaneous absorption of nicardipine hydrochloride.

The liquid components described above are uniformly mixed.

When using middle chain fatty acid glyceride and/or sorbitan middle chain fatty acid ester, it is preferred to use propylene glycol together therewith.

Although nicardipine hydrochloride has low solubility in the above individual components of [b], the mixed liquid used according to the invention can increase its solubility and dispersability, and by dispersing the resulting composition [b] in a transdermal formulation base good percutaneous absorption of nicardipine hydrochloride is obtainable.

Further, in the case of using the middle chain fatty acid glyceride or the sorbitan middle chain fatty acid ester there is a heterogeneous range over which they do not mix uniformly, but by adding nicardipine hydrochloride to a mixture of these components in the heterogeneous range an unexpectedly uniform nicardipine hydrochloride solution can be obtained, whereby the dissolution ranges of the above-described liquid components can be enlarged and satisfactory preparation of the transdermal formulation with percutaneous absorption can be obtained.

There is no particular restriction as to the

proportion of the liquid components to nicardipine hydrochloride, provided that it is enough to uniformly dissolve or suspend nicardipine hydrochloride in the mixture of these components and can keep the form of a transdermal formulation when the mixture is compounded with a transdermal formulation base. The proportion of liquid components depends upon the kind of base to be used and on any other compounding components present, but it is preferable to control the amount of mixed liquid according to the proportion of nicardipine hydrochloride - the latter usually being 1 to 20 w/w% based on the total final formulation.

If the concentration of nicardipine hydrochloride is high, it can crystallize when stored for a long period of time. To inhibit this, compounding with polyoxyethylene-hardened castor oil, neopentyl glycol di-2-ethylhexanoate, propylene glycol didecanoate, etc., is effective. A suitable proportion of the crystallizing inhibitor is 0.5 to 10 w/w%, preferably 1 to 5 w/w%, of the total final formulation.

The transdermal formulations of this invention may further contain auxiliary compounds for stabilization and pH control.

To assist uniform dispersion of nicardipine hydrochloride in the base, an emulsifier may be used.

As the emulsifier, nonionic surface active agents, for example, glycerol higher fatty acid esters such as glyceryl monostearate (Nikkol MGS-B, trade name, made by Nikko Chemical Co., Ltd.), polyglycerol fatty acid ester (Nikkol Decaglyn, trade name, made by Nikko Chemical Co., Ltd.), etc.; polyoxyethylene sorbitan monostearate (Tween 60, made by Kao Atlas Co., Ltd.); sorbitan higher fatty acid esters such as sorbitan monostearate (Span 60, trade name, made by Kao Atlas Co., Ltd.), etc,; polyoxyethylene monolaurate (Nikkol MYL-10, trade name, made by Nikko Chemical Co., Ltd.); polyoxyethylene lauryl ether (Nikkol BL-9EX, trade name, made by Nikko Chemical Co., Ltd.); polyoxyethylene polyoxypropylene copolymer (Pluronic F68, trade name, made by Asahi Denka Kogyo K. K.), etc., can be used. A suitable proportion of the emulsifier is 0.5 to 10 w/w%, based on the total final formulation.

There is no particular restriction as to the base for use in this invention but usually white petrolatum, plastibase , silicone oils, etc., are preferred. By suitably selecting the base and other compounding components, transdermal formulations such as ointments, tapes, patches, cataplasms, etc., can be prepared by conventional methods.

For example, an ointment of this invention can be prepared by dissolving nicardipine hydrochloride in a

mixed liquid of a urea and at least one of propylene glycol, monohydric alcohols having 2 to 4 carbon atoms, lactic acid, thioglycol, middle chain fatty acid glycerides and sorbitan middle chain fatty acid esters, gradually adding the solution to a mixture of an ointment base and an emulsifier (which has been melted at 60°C and then cooled to room temperature) until the resultant mixture becomes uniform, and sufficiently kneading the mixture. A crystal deposition inhibitor can be added to the ointment thus obtained followed by uniform mixing. As the base which is used for the preparation of the ointment, the above-described oily bases are suitable.

To prepare a patch, a carrier such as a nonwoven fabric, paper, cloth, plastics film or sheet, etc., can be impregnated with a liquid obtained by dissolving or suspending nicardipine hydrochloride in an aqueous solution of a urea and at least one of propylene glycol, monohydric alcohols having 2 to 4 carbon atoms, lactic acid, thioglycol, middle chain fatty acid glycerides and sorbitan middle chain fatty acid esters (or the aforesaid liquid is gelled and spread over a flexible metal foil) and, if necessary, an adhesive layer is formed thereon.

In this case, an antiseptic (e. g., paraben, etc.,), a humidity retaining agent (e. g., glycerol, etc.,), a

suspending agent (e. g., silicic anhydride, Aerosil, trade name), etc., may be compounded.

Percutaneous absorption and storage stability tests were conducted on transdermal formulations of this invention as follows.

(1) Percutaneous absorption test in guinea pigs.

Guinea pigs (weighing about 400 g), with backs shaved by an electric clipper and a depilatory cream, were used. About 1 g of each of the ointments obtained in Examples 1, 2, 3, 9, 10, 11, 12, 13 and 14 described hereinbelow placed in a plastic container (about 10 $cm^2$ in area) was stuck to the shaved skin of respective guinea pigs and after covering the ointment with parafilm, it was fixed by a gum tape. 5 hours after the transdermal administration of the ointment, blood was collected and the plasma concentration of nicardipine hydrochloride was measured according to Higuchi et al's method ("Journal of Chromatography", 110, 301(1975)). The results are shown below.

Result

| Test ointment | Plasma concentration (ng/ml) of nicardipine hydrochloride |
|---|---|
| Example 1 | 16.3 |
| " 2 | 29.0 |
| " 3 | 64.0 |
| " 4 | 70.3 |
| " 9 | 79.7 |
| " 10 | 57.5 |
| " 11 | 42.0 |
| " 12 | 73.8 |
| " 13 | 60.0 |
| " 14 | 65.0 |
| Reference sample * | 5.7 |

(*): The ointment used in the comparison test was prepared by melting 186.4 g of white vaseline and 9.1 g of Nikkol MGS-B at about 60°C, directly adding 4.5 g of nicardipine hydrochloride to the molten mixture in a kneader, and kneading the mixture well.

(2) Stability test

(crystal deposition of nicardipine hydrochloride in ointments):

The extent of the crystal deposition of nicardipine hydrochloride was observed, using a polarising microscope, in the ointments prepared in Examples 13 to 16 both directly after their preparation and after storing for one month at room temperature.

The results show that the crystal deposition was very slight in Examples 14 to 16 as compared to Example 13.

The following examples are intended to illustrate the present invention but not to limit in any way.

Example 1

After melting 159.1 g of white petrolatum and 9.1 g of Nikkol MGS-B at about 60$^{\circ}$C, the molten mixture was transferred into a kneader. Then, a solution of 4.5 g of nicardipine hydrochloride in 27.3 g of a mixed solvent of urea/pure water/ethanol (1 : 1 : 1 by weight ratio) was gradually added to the mixture with stirring and the resultant mixture was kneaded to provide an ointment.

Example 2

After melting 150 g of white petrolatum and 9.1 g of Nikkol MGS-B at about 60$^{\circ}$C, the molten mixture was transferred into a kneader and then by following the same procedure as Example 1 using a solution of 4.5 g of nicardipine hydrochloride in 36.4 g of a mixed solvent of urea/pure water/ethanol/propylene glycol (1 : 1 : 1 : 1 by weight ratio), an ointment was obtained.

Example 3

After melting 159.1 g of white petrolatum and 9.1 g of Nikkol MGS-B, the molten mixture was transferred into a kneader and then, by following the same procedure as Example 1 using a solution of 4.5 g of nicardipine hydrochloride in 27.3 g of a mixed solvent of urea/pure water/lactic acid (1 : 1 : 1 by weight ratio), an ointment was obtained.

Example 4

After melting 150 g of white petrolatum and 9.1 g of Nikkol MGS-B at about 60°C, the molten mixture was transferred into a kneader and then by following the same procedure as Example 1 using a solution of 4.5 g of nicardipine hydrochloride in 36.4 g of a mixed solvent of urea/pure water/lactic acid/propylene glycol (1 : 1 : 1 : 1 by weight ratio), an ointment was obtained.

Example 5

After melting 150 g of white petrolatum and 9.1 g of Nikkol MGS-B at about 60°C, the molten mixture was transferred into a kneader and then by following the same procedure as Example 1 using a solution of 4.5 g of nicardipine hydrochloride in 36.4 g of a mixed solvent of urea/pure water/lactic acid/ethanol (1 : 1 : 1 : 1 by weight ratio), an ointment was obtained.

Example 6

After melting 163 g of white petrolatum and 9.1 g of Nikkol MGS-B at about 60°C, the molten mixture was transferred into a kneader and then by following the same procedure as Example 1 using a solution of 4.5 g of nicardipine hydrochloride in 22.8 g of a mixed solvent of thioglycol/urea/pure water (1 : 1 : 0.5 by weight ratio), an ointment was obtained.

Example 7

A mixture of 40 g of diisopropyl adipate, 45.5 g

of a middle chain fatty acid triglyceride (ODO, trade name, made by Nisshin Seiyu K. K.), 40 g of isopropyl myristate, and 10 g of glyceryl monostearate (Nikkol MGS-B) was heated to about 50°C to provide liquid A.

Also, 4.5 g of nicardipine hydrochloride was dissolved in 40 g of a mixed liquid of lactic acid/-urea/pure water/propylene glycol (1 : 1 : 1 : 1 by weight ratio) to provide liquid B.

Liquid A was gradually added to 20 g of Aerosil (made by Nippon Aerosil K. K.) in a kneader followed by kneading and liquid B was gradually added thereto followed by sufficient kneading to provide an ointment.

Example 8

A mixture of 27.5 parts of pure water, 5.0 parts of concentrated glycerol and 5.0 parts of gelatin was heated to 50°C to provide a solution, 20.0 parts of kaolin was added to the foregoing solution followed by kneading, 2.5 parts of sodium polyacrylate and 15.0 parts of concentrated glycerol were added thereto followed by kneading, 1.0 part of polyoxyethylene sorbitan monostearate and 7.0 parts of liquid paraffin were added thereto followed by kneading, and then 10.0 parts of a previously prepared nicardipine hydrochloride solution [a solution of 0.5 part of nicardipine hydrochloride in 9.5 parts of a mixed solvent of urea/pure water/lactic acid/propylene glycol (1 : 1 : 1 : 1 by weight ratio)] and 7.0 parts of a

natural rubber latex were added thereto followed by uniformly kneading. The kneaded mixture thus obtained was spread over flannel and after sticking a plastics film to the surface thereof, the fabric was cut to predetermined size.

Example 9

After melting 148.5 g of white petrolatum and 9.0 g of Nikkol MGS-B at about 60°C, the molten mixture was transferred into a kneader. Then a solution of 4.5 g of nicardipine hydrochloride in a mixed liquid of 11 g of Nikkol MGK, 9.0 g of urea, 9.0 g of pure water, and 9.0 g of propylene glycol was gradually added thereto with stirring and the mixture was kneaded to provide an ointment.

Example 10

By following the same procedure as Example 9 except that 11 g of Sunsoft No. 700p-2 (made by Taiyo Kagaku K. K.) was used in place of 11 g of Nikkol MGK, an ointment was obtained.

Example 11

By following the same procedure as Example 9 except that 6.0 g of Nikkol MGK and 5.0 g of Nikkol HCO-60 were used in place of 11 g of Nikkol MGK, an ointment was obtained.

Example 12

After melting 158.3 g of white petrolatum and 9.0 g of Nikkol MHS-B at about 60°C, the molten mixture was transferred into a kneader. Then, a solution of 4.5 g

nicardipine hydrochloride . in a mixture of 11 g of Nikkol MGK, 4.8 g of urea, 3.2 g of pure water, 8.2 g of propylene glycol, and 1.0 g of lactic acid was gradually added to the molten mixture with stirring and the resultant mixture was kneaded to provide an ointment.

Example 13

After melting 148.5 g of white petrolatum and 9.0 g of Nikkol MGS-B at about 60°C, the molten mixture was transfered into a kneader. Then, a solution of 4.5 g of nicardipine hydrochloride dissolved in a mixture of 11 g of Nikkol SK-10 (sorbitan monocaprylate, made by Nikko Chemicals Co., Ltd.), 9.0 g of urea, 9.0 g of pure water, and 9.0 g of propylene glycol under heating was gradually added to the molten mixture and the resultant mixture was kneaded to provide an ointment.

Example 14

After melting 139.5 g of white petrolatum and 9.0 g of Nikkol MGS-B at about 60°C, the molten mixture was transferred into a kneader. Then, a solution of 4.5 g of nicardipine hydrochloride dissolved in a mixture of 11 g of Nikkol SK-10, 9.0 g of urea, 9.0 g of pure water, and 9.0 g of propylene glycol under heating was gradually added to the molten mixture with stirring and finally 9.0 g of propylene glycol didecanoate was added thereto followed by kneading to provide an ointment.

Example 15

By following the same procedure as Example 14

except that neopentyl glycol di-2-ethylhexanoate was used in place of propylene glycol didecanoate, an ointment was prepared.

Example 16

By following the same procedure as Example 14 except that Nikkol HCO-60 was used in place of propylene glycol didecanoate, an ointment was prepared.

CLAIMS:

1. A medicament composition suitable for transdermal applications, the composition comprising nicardipine hydrochloride dissolved or suspended in a liquid containing a urea and at least one compound selected from propylene glycol, monohydric alcohols having 2 to 4 carbon atoms, lactic acid, thioglycol, middle chain fatty acid glycerides and sorbitan middle chain fatty acid esters.

2. A composition according to claim 1 wherein the said liquid includes ethanol.

3. A composition according to claim 1 or 2 wherein the middle chain fatty acid glyceride component is selected from mono- and di-glycerides of fatty acids having 6 to 12 carbon atoms.

4. A composition according to claim 1,2 or 3 wherein the sorbitan middle chain fatty acid ester component is selected from mono- and di-esters of fatty acids having from 6 to 12 carbon atoms.

5. A composition according to any preceding claim wherein the said liquid includes urea.

6. A composition according to any preceding claim wherein the said liquid includes water.

7. A transdermanl formulation of nicardipine hydrochloride which comprises a composition according to any preceding claim compounded with a transdermal formulation base.

8. A composition or formulation according to any preceding claim containing a crystallization inhibitor for nicardipine hydrochloride.

9. A composition or formulation according to claim 8 wherein the crystallization inhibitor is at least one compound selected from propylene glycol didecanoate, neopentyl glycol, di-2-ethylhexanoate, and polyoxyethyelne-hardened castor oil derivatives.

10. A composition or formulation according to any preceding claim on a substrate carrier layer, e.g. of fabric, paper, plastics or metal.